# EUROPÄISCHE PATENTSCHRIFT

(11) **EP 0 642 365 B1**
(45) Veröffentlichungstag und Bekanntmachung des Hinweises auf die Patenterteilung: **01.04.1998**
(21) Anmeldenummer: 93910013.7
(22) Anmeldetag: 18.05.1993
(51) Int. Cl.: A61M 5/315

(54) **SPRITZE**
SYRINGE
SERINGUE

(30) Priorität: 21.05.1992 DE 9206878 U; 13.06.1992 DE 4219502
(43) Veröffentlichungstag der Anmeldung: 15.03.1995
(73) Patentinhaber: WASKOENIG, Wilhelm, E-04720 Aguadulce (ES)
(72) Erfinder: WASKOENIG, Wilhelm, E-04720 Aguadulce (ES)
(74) Vertreter: Stoffregen, Hans-Herbert, Dr. Dipl.-Phys.
(86) Internationale Anmeldenummer: EP9301237
(87) Internationale Veröffentlichungsnummer: WO9323097

(56) Entgegenhaltungen:
- CH-A- 286 277
- FR-A- 1 053 583
- FR-A- 1 108 413

## Beschreibung

Die Erfindung bezieht sich auf eine Spritze nach dem Oberbegriff des Anspruchs 1.

Es stehen verschiedene Spritzentypen zur Verfügung, bei der zur Lokalisierung des Epiduralraumes die Widerstandsveränderungen beim Bewegen des Kolbens (sogenannte loss of resistance Spritze) genutzt wird. Entsprechende aus einem Kunststoffzylinder und -kolben bestehende Spritzen zeigen eine Gemeinsamkeit darin, daß dem Vortrieb des Spritzenkolbens wenig Widerstand entgegengesetzt wird, wodurch ein Druckwechsel unmittelbar übertragen und damit feststellbar wird.

Ferner sollte die Eigenschaft gegeben sein, daß während des Verschiebens des Kolbens die Reibung zwischen diesem und der Zylinderinnenwandung über den gesamten Kolbenhub konstant ist, so daß eine gleichförmige Kolbenbewegung durch geringen Druck auf den Kolben selbst erzielt werden kann.

Eine diese Eigenschaften aufweisende aus Kunststoff bestehende Spritze, die zur einmaligen Verwendung bestimmt ist, wurde von Portex entwickelt. Hierbei handelt es sich um eine dreiteilige Spritze, deren Hauptvorteil in der hohen Maßgenauigkeit von Kolben und Zylinder lag, wodurch eine gute Druckübertragung bei geringer Reibung ermöglicht wurde. Durch die Kolbenform bedingt konnte die diesbezügliche Spritze jedoch nur beim Injizieren wirkungsvoll arbeiten, da beim Aspirieren die Dichtung zwischen Kolben und Zylinderinnenwandung nur ungenügend Wirkung entfaltete, so daß beim Zurückziehen des Kolbens der aufgebaute Unterdruck nicht ausreichte, um Flüssigkeit aufzusaugen.

Eine weitere, die eingangs beschriebenen Eigenschaften aufweisende Spritze ist von Braun entwickelt worden. Allerdings zeigte die diesbezügliche Spritze bei der Aspiration die gleichen Nachteile wie die Portex-Spritze, d. h., daß ein Einsatz zur Aspiration grundsätzlich nicht möglich war.

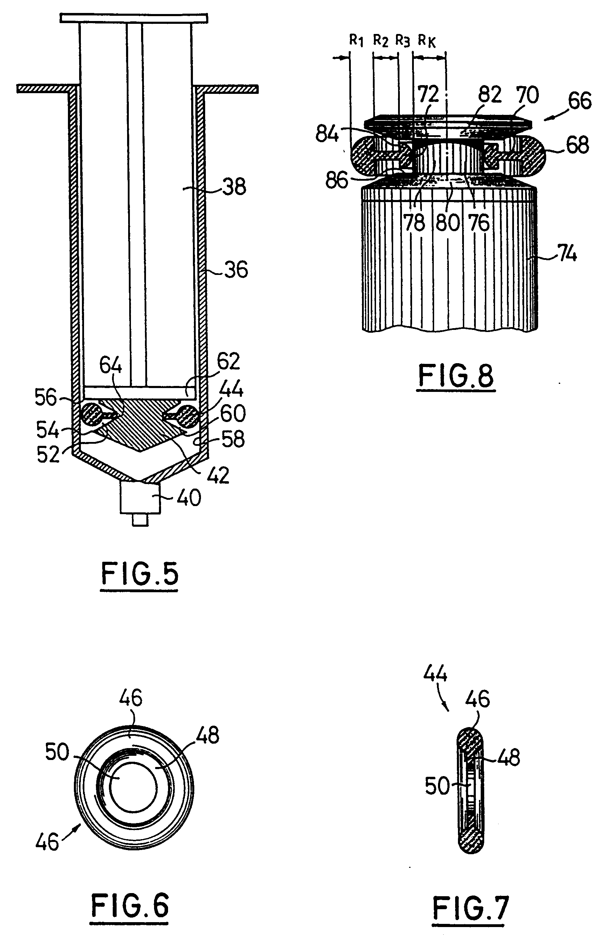

Eine gattungsbildende Spritze ist der FR 1 048 267 zu entnehmen. Die der Fig. 8 zu entnehmende Dichtung besteht aus drei Abschnitten, von denen ein innerster im Schnitt eine rechteckförmige Geometrie aufweist und sich über die gesamte Breite einer die Dichtung aufnehmenden Nut des Spritzenkolbens erstreckt. Nach der Fig. 6 kann die Dichtung aus einem großen äußeren Abschnitt rechteckförmigen Querschnitts, der sich an der Zylinderinnenwandung der Spritze anlegt, und einem kleinen stegförmigen inneren Abschnitt bestehen. Die entsprechende Dichtung ist recht starr und bietet keine hinreichende Dichtung bein Injizieren bzw. Asperieren.

Aus der CH 286 277 ist eine Injektionsspritze mit einer Dichtung bekannt, die sich aus einem äußeren wulstartigen sich flächig an der Zylinderinnenwandung der Spritze anlegenden Abschnitt und einem inneren membranartigen Abschnitt zusammensetzt, der den Spritzenkolben umgibt.

Eine Dichtung für eine Injektionsspritze gemäß der FR 1 108 413 besteht aus einem im Schnitt tropfenförmigen äußeren Abschnitt, der über einen Steg in einen inneren wulstartigen Abschnitt übergeht. Sowohl der äußere als auch der innere Abschnitt liegt flächig an der Zylinderinnenwandung bzw. dem Kolben der Spritze an.

Aus der US 4,632,672 ist eine Spitze mit einem in einer umlaufenden Nut des Spritzenzylinders angeordnetem O-Ring bekannt.

In der DE 4 566 602 wird eine Injektionsspritze beschrieben, die aus Kunststoff besteht und die eingangs beschriebene Nachteile aufweist.

Eine Flachdichtung dient nach der DE 2 024 117 zum Abdichten eines Kolblens genenüber einem Zylinder einer aus Kunststoff bestehenden Injektionsspritze.

Weitere Spritzen sind der US 11 54 677, US 25 78 814, EP 0 102 070 A2, FR 24 06 988, GB 11 79 487 und DE 24 51 398 A1 zu entnehmen.

Der vorleigenden Erfindung liegt das Problem zugrunde, eine Spritze der eingangs genannten Art derart weiterzubilden, daß sowohl eine Verwendung zum Injizieren als auch zum Aspirieren möglich ist, wobei gleichzeitig geringe Reibungsverluste beim Verschieben des Kolbens auftreten sollen.

Das Problem wird erfindungsgemäß durch die Maßnahmen des Anspruchs 1 gelöst.

Dadurch, daß sich die Dichtung aus dem beim Injizieren bzw. Aspirieren die Dichtwirkung hervorrufenden ringförmigen äußeren Abschnitte, der einer halben O-Ring-Dichtung gleichkommt, und dem mittleren flexiblen haut- bzw. membranartigen Abschnitt, der durch den gleichfalls einer halben O-Ring-Dichtung gleichkommenden inneren Abschnitt stabilisiert wird und den Kolben dicht umgibt, zusammensetzt, ergibt sich der Vorteil, daß in Ruhelage des Kolbens ein möglicherweise auftretendes Hinterwandern der Dichtung unterbleibt. Der innere Abschnitt der Dichtung ist dabei derart ausgebildet, daß Rückstellkräfte im wesentlichen nicht auftreten, wodurch andernfalls die Reibungskräfte beim Verschieben des Kolbens beeinflußt würden.

Vorzugsweise ist die radiale Erstreckung des äußeren Abschnitts gleich oder größer als die des inneren Abschnitts, also des membranartigen Abschnitts einschließlich dessen Verstärkung. Bei entsprechender bevorzugter Form der Dichtung sollte der sich zwischen den wulstartigen, an der Zylinderinnenwandung bzw. an dem Kolben dichtend anliegenden Abschnitten sich erstreckende haut- oder membranartige Abschnitte eine radiale Erstreckung aufweisen, die in etwa der des äußeren Abschnitts, jedoch doppelt so groß wie die des inneren Abschnitts ist.

Die Dichtung selbst bzw. deren mit den Kolbenabschnitten bzw. der Zylinderinnenwandung wechselwirkende Abschnitte sind bei unbenutzter Spritze mit Spiel zwischen den Abschnitten des Kolbens angeordnet, wohingegen eine Abdichtung zwischen der Zylinderinnenwandung und einem der Abschnitte beim Injizieren bzw. Aspirieren erfolgt.

Mit der erfindungsgemäßen Spritze treten nur geringe Reibungsverluste zwischen Kolben und Zylinderinnenwandung auf, da der Kolben aufgrund der Dichtung nur einen Minimalkontakt mit der Innenwandung erreicht. Sowohl beim innenliegenden haut- oder membranartigen Abschnitt als auch dann, wenn dieser von der als halbzylinderförmiger Ring ausgebildeten Verstärkung begrenzt ist, erfolgt stets eine im wesentlichen linienförmige Umschließung des Kolbens. Eine entsprechende im wesentlichen linienförmige Abdichtung erfolgt auch zwischen der Zylinderinnenwandung und dem äußeren Abschnitt der Dichtung. Folglich ist die aufzuwendende Kraft zum Verschieben des Kolbens geringer als bei den bekannten Spritzen, da bei diesen zum Vermeiden von Druckverlusten ein stärkerer Druck auf die Seitenwände ausgeübt werden muß.

Dadurch, daß sich die Dichtung selbsttätig an einem der Abschnitte, und zwar bei der Injektion an dem der Kolbenstirnseite abgewandten Abschnitt und der Kolbeninnenwandung selbsttätig anlegt, wobei bei zunehmendem Druck eine automatische Erhöhung der Dichtung erfolgt, ist eine hohe Sicherheit bei der Nutzung der Spritze gewährleistet.

Gleiche Überlegungen gelten beim Aspirieren, da mit zunehmendem Unterdruck eine zunehmende Dichtung zwischen dem stirnseitig naheliegenden Abschnitt und der Zylinderinnenwandung bewirkt wird.

Bei der Dichtung sollte ein Material verwendet werden, dessen Dichte zwischen 2 und 75, vorzugsweise zwischen 30 und 50 Shore liegt. Dadurch ist beim Injizieren bzw. Aspirieren eine Verformung der Dichtung sichergestellt, die mit zunehmender Druckeinwirkung eine höhere Dichtwirkung hervorruft.

Die erfindungsgemäße Spritze ist folglich bei geringen Reibungsverlusten gleichermaßen gut zum Injizieren und Aspirieren verwendbar, und zwar aufgrund der mit Spiel freibeweglichen Anordnung der Dichtung zwischen den Kolbenabschnitten bzw. den Begrenzungen der Aufnahme, wodurch die Möglichkeit geschaffen wird, daß sich die Dichtung, je nachdem ob die Spritze zum Injizieren oder zum Aspirieren benutzt wird, wahlweise zwischen einem der Kolbenabschnitte und der Zylinderwendung dichtend anlegt.

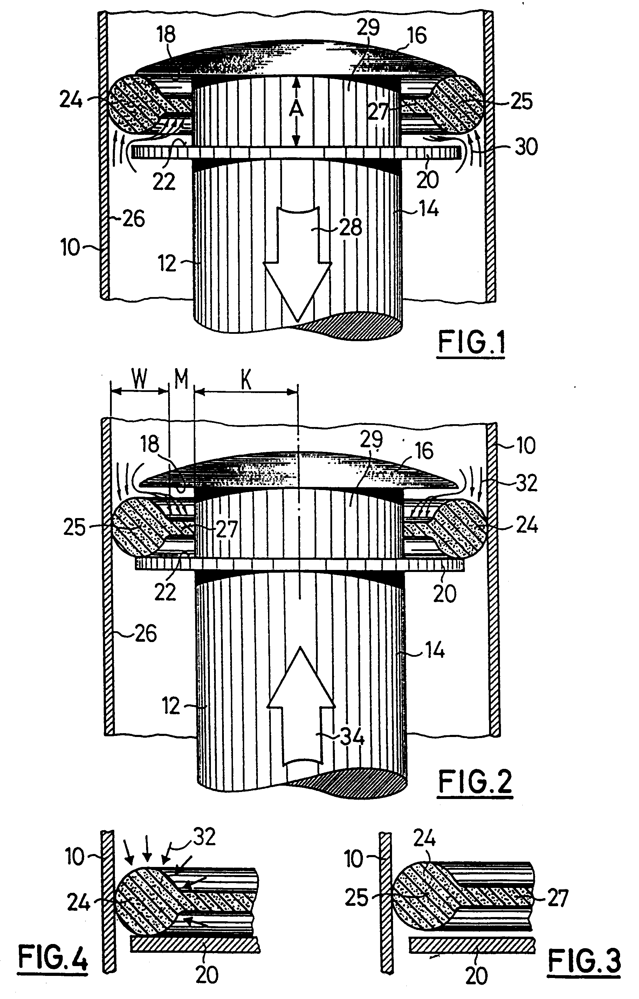

Weitere Einzelheiten, Vorteile und Merkmale der Erfindung ergeben sich nicht nur aus den Ansprüchen, sondern auch aus der nachfolgenden Beschreibung eines der Fig. 8 zu entnehmenden Ausführungsbeispiels, welches eine der Lehre der Ansprüche entsprechenden Spritze dargestellt, wobei die den Fig. 1 - 7 zu entnehmenden Ausführungsbeispiele, die nicht im beanspruchten Schutzbereich fallen zur Erläuterung grundsätzlicher Merkmale einer zum Injizieren und Aspirieren geeigneten Spritze dienen.

Es zeigen:
- Fig.1: einen Ausschnitt eines ersten Ausführungsbeispiels einer Spitze während des Aspirierens,
- Fig.2: einen Ausschnitt der Spritze nach Fig. 1 während des Injizierens,
- Fig.3: eine verwendete Dichtung bei einer Spritze in Ruhestellung,
- Fig.4: die verwendete Dichtung bei arbeitender Spritze,
- Fig. 5: eine zweite Ausführungsform einer Spritze,
- Fig. 6: eine Draufsicht auf eine Dichtung,
- Fig. 7: einen Schnitt durch die Dichtung nach Fig. 6 und
- Fig. 8: einen Ausschnitt eines Spritzenkolbens mit doppelhantelförmiger Dichtung gemäß Anspruch 1.

In den Fig. 1 und 2 ist ein Ausschnitt einer vorzugsweise aus Kunststoff bestehenden Einmalspritze dargestellt, mit der sowohl injiziert als auch aspiriert werden kann. Die Spritze umfaßt einen Hohlzylinder (10) mit in diesen, verschiebbar angeordneten Kolben (12). Der Kolben (12) weist einen zylinderischen Basiskörper (14) auf, dessen Außendurchmesse kleiner als der Innendurchmesser des Zylinders (10) ist. Nadelseitig besitzt der Kolben (12) einen Stirnabschnitt (16), der außenseitig gewölbt ist und vorzugsweise der Geometrie eines Kugelabschnittes folgt. Der Stirnabschnitt (16) erstreckt sich radial über den Basiskörper (14) des Kolbens (12) hinaus, wobei jedoch der maximale Außendurchmesser kleiner als der Innendurchmesser des Zylinders (10) ist.

Die von dem Basiskörper (14) ausgehende innere Fläche (18) des Stirnabschnittes (16) ist plan ausgebildet, wie die Fig. 1 und 2 verdeutlichen.

Im Abstand zu der Fläche (18) verläuft ein sich gleichfalls radial über den Basiskörper (14) des Kolbens (12) erstreckender scheibenförmiger Abschnitt (20), dessen Außendurchmesser gleichfalls geringer als der Innendurchmesser des Zylinders (10) ist. Der lichte Abstand zwischen den aneinander zugewandten und parallel zueinander verlaufenden Flächen (18) und (22) des Stirnabschnittes (16) und des Abschnitts (20) ist in der Fig. 1 mit A bezeichnet.

Der Kolben (12) bzw. die von diesem ausgehenden Abschnitte (16) und (20) weisen einen Durchmesser auf, der geringfügig kleiner als der Innendurchmesser des Zylinders (10) ist, so daß ein unmittelbarer Kontakt ausgeschlossen ist.

Zwischen dem Stirnabschnitt (16) und dem Abschnitt (20), also zwischen den Flächen (18) und (22) ist eine Dichtung (24) angeordnet, der in axialer Richtung des Kolbens (12) verlaufende Erstreckung kleiner als der lichte Abstand A ist. Der Außendurchmesser der Dichtung (24) ist zumindest gleich dem Innendurchmesser des Zylinders (10).

Die Dichtung (24) setzt sich dabei aus einem äußeren einem O-Ring gleichkommenden Abschnitt (25) und einem inneren haut- oder membranartigen Abschnitt (27) zusammen, der den Kolbenabschnitt (29) dicht umschließt.

Bei einer nicht arbeitenden Spritze ist die Dichtung (24) mit geringstmöglichem Spiel freibeweglich zwischen den Abschnitten (16) und (20) angeordnet. Dabei erfolgt nicht zwingender Weise eine Abdichtung zwischen der Dichtung (24) und einem der Abschnitte (16) oder (20) und der Zylinderinnenwandung (26). Dies soll anhand der Fig. 3 verdeutlicht werden.

Wird die Spritze zum Aspirieren benutzt (Fig. 1), wird der Kolben (12) von der Spritzennadel weggezogen, also in Richtung des Pfeils (28) bewegt. Durch diese Bewegung wird die Dichtung (24) gezwungen, sich zwischen der Fläche (18) des Stirnabschnitts (16) und der Innenwandung (26) des Zylinders (10) anzulegen. Hierdurch erfolgt eine Abdichtung, die mit stärker werdender Kolbenbewegung erhöht wird. Gleichfalls wirkt auf die Dichtung (24) ein Druck (durch die Pfeile 30 angedeutet), wodurch die Dichtwirkung erhöht wird. Folglich ist ein problemloses Aspirieren und damit Absaugen von Flüssigkeit möglich. Gleichzeitig sind jedoch nur geringe Reibungswiderstände zu überwinden, da die thorische Dichtung (24) nur eine geringe Auflagefläche (linienförmige Berührung) an der Zylinderinnenwandung (26) benötigt, um wirksam zu sein. Dies schließt jedoch nicht aus, daß von dem Basiskörper (14) Führungsvorsprünge ausgehen, die an der Innenwandung (26) abstützbar sind.

Gleichfalls erfolgt eine linienförmige Bewulstung zwischen dem inneren Abschnitt (27) und dem Kolbenabschnitt (29). Gegebenenfalls kann das an dem Kolbenabschnitt anliegende Ende des Dichtungsabschnitts (27) verjüngt bzw. angefast sein.

Aber nicht nur beim Aspirieren, sondern auch beim Injizieren erfolgt ein sicheres Abdichten über die Dichtung (24), und zwar - wie die Fig. 2 verdeutlicht - zwischen der Innenwandung (26) und dein Abschnitt (20). Durch den sich aufbauenden Innendruck in der Spritze erfolgt gleichzeitig eine Druckeinwirkung auf die Dichtung (24) (Pfeile 32), die eine erhöhte Abdichtung sicherstellt. Dies soll auch anhand der Fig. 4 verdeutlicht werden.

Beim Injizieren wird der Kolben (12) in Richtung der Spritzennadel, also in Richtung des Pfeils (34) bewegt.

Auch beim Injizieren tritt nur ein geringer Reibungswiderstand auf, da grundsätzlich die Dichtung (24) allein entlang der Innenwandung (26) gleitet, also Reibungswiderstände zwischen den, Kolben (12) und Zylinder (10) im eigentlichen Sinne nicht überwunden werden müssen.

In den Fig. 5 bis 7 ist ein weiteres Ausführungsbeispiel einer Spritze dargestellt.

Wie bei dem Ausführungsbeispiel der Fig. 1 bis 4 ist in einem Zylinder (36) ein Kolben (38) verschiebbar angeordnet, der stirnseitig, also im Bereich seinem dem Spritzenkopf (40) zugewandtem Ende (42) geschnitten dargestellt ist.

Der Durchmesser des Kolbens (38) ist erkennbar geringer als der Innendurchmesser des Zylinders (36), so daß ein unmittelbarer Kontakt ausgeschlossen ist. Dennoch erfolgt eine sichere Abdichtung zwischen dem Kolben (38) und dem Zylinder (36) sowohl beim Injizieren als auch beim Aspirieren.

Hierzu ist im Bereich des vorderen Endes (42) des Kolbens (38) eine Dichtung (44) vorgesehen die sich im eigentlichen Sinne aus einem O-Ring (46) als äußeren umlaufenden Wulst und einem inneren Abschnitt (48) zusammensetzt, der eine zentrale Durchbrechung (50) aufweist. Der Abschnitt (48) kann membranartig ausgebildet sein. Auch kann der Abschnitt (48) durch eine von dem äußeren Abschnitt (46) ausgehende umlaufende Lippe gebildet sein.

Die entsprechend ausgebildete Dichtung (44), die im Schnitt eine hantelförmige Geometrie mit zentraler Durchbrechung zeigt, ist im Bereich (42) des Kolbens (38) in einer Aufnahme (52) festgelegt, die durch eine umlaufende und im Schnitt V-förmige Vertiefung gebildet wird.

Die radial sich erstreckenden Begrenzungen der Vertiefung, also die äußeren Ränder (54) und (56) der Aufnahme (52) enden im Abstand zur Innenwandung (58) des Zylinders (36).

Zur Erzielung einer Abdichtung weist die Dichtung (44) einen maximalen Durchmesser auf, der gleich oder größer als der Innendurchmesser der Innenwandung (58) ist.

Im Bereich der wulstartigen Erstreckung (48) weisen die Begrenzungswandungen (60) und (62) der Aufnahme (52) einen Abstand auf, der größer als der Durchmesser des Abschnittes (46) der Dichtung (44) ist. Folglich ist die Dichtung (44) frei beweglich in der Aufnahme (52) angeordnet. Diese freie Beweglichkeit wird grundsätzlich auch nicht durch den membran- oder hautförmigen Abschnitt (48) der Dichtung (44) eingeengt, der sich dicht um den Boden (64) der Aufnahme (52) anlegt, um ein Hinterwandern der Dichtung (44) bei ruhendem Kolben (38) auszuschließen. Der Abschnitt (48) ist dabei derart in seiner Eigensteifigkeit ausgebildet, daß Rückstellkräfte nicht oder nicht merklich auftreten, so daß folglich durch den Abschnitt (48) auch keine erhöhten Reibungskräfte verursacht werden können.

Die radiale Erstreckung W des äußeren Abschnitts (46) der Dichtung (44) ist in etwa doppelt so groß wie die radiale Erstreckung M des inneren membranartigen Abschnitts (48), der den Boden (64) der im Schnitt V-förmigen Aufnahme (52) dicht umgibt. Ferner sollte der Durchmesser K im Bereich des Bodens (64) des Kolbens (38) gleich der gesamten radialen Erstreckung des äußeren Abschnitts (46) der Dichtung (44) sein, also 2 W ≈ K.

Durch die Ausbildung und Anordnung der Dichtung (44) des den Fig. 5 bis 7 zu entnehmendem Ausführungsbeispiels ergeben sich die gleichen Wirkungen wie bei der Spitze der Fig. 1 bis 4. Dies bedeutet, daß beim Injizieren der umlaufende Wulst (46) zwischen der Begrenzung (62) und der Zylinderinnenwandung (58) und beim Aspirieren zwischen der Begrenzung (60) und der Zylinderinnenwandung (58) dichtend anliegt.

Der Fig. 8 ist ein Ausschnitt einer der Erfindung entsprechenden Spritze zu entnehmen. So ist eine Dichtung (66) vorgesehen, die aus einem äußeren halbzylinderischen Ring (68), einem stegförmigen kreisringförmigen mittleren Abschnitt (70) und einem inneren Abschnitt (72) in Form eines halbzylinderischen Rings besteht. Die Dichtung (66) umgibt einen Kolben (74) in einer Aufnahme (76) dicht und ist gleichzeitig dichtend an die Innenwandung eines nicht dargestellten Zylinders einer Spritze angelegt.

Die Aufnahme oder Nut (76) besteht aus einem in Längsrichtung des Kolbens (74) verlaufenden ebenen Boden (78), der von radial, und senkrecht zur Längsachse des Kolbens (74) sich erstreckenden Wandungen (84) und (86) begrenzt ist. An diesem im Schnitt rechteck- oder quadratförmigen Abschnitt schließt sich ein sich konisch nach außen vergrößernder Abschnitt an, der von Seitenwandungen (80) und (82) begrenzt wird. Der Öffnungswinkel zwischen den Seitenwandungen (80) und (82) sollte zwischen 30° und 50°, vorzugsweise bei in etwa 40° liegen.

Mit anderen Worten setzt sich die Aufnahme (76) im Schnitt aus äußeren trapezförmigen und inneren quadrat- bzw. rechteckförmigen Abschnitten zusammen.

Die axiale Ersteckung des inneren von den Seitenwandungen (84) und (86) begrenzenden Abschnitts der Aufnahme (76) ist größer als die des inneren Abschnitts (72) der Dichtung (66). Auch in radialer Erstreckung ist der innere Abschnitt der Aufnahme (76) größer. Dies bedeutet, daß der innere mit seiner konvexen Seite an dem Boden (78) anliegende innere Abschnitt (72) der Dichtung (66) vollständig innerhalb des im Schnitt rechteck- bzw. quadratförmigen Abschnitts der Aufnahme (76) liegt.

Der äußere, gleichfalls einem halben O-Ring gleichkommende Abschnitt (68), der sowohl in axialer als auch in radialer Richtung eine größere Erstreckung als der innere Abschnitt (72) aufweist, wird bereichweise von dem im Schnitt trapezförmigen äußeren Abschnitt der Aufnahme (76) umgeben, wobei jedoch eine axiale Erstreckung vorliegt, die sicherstellt, daß der Abschnitt (68) der Dichtung (66) bei ruhender Spritze beabstandet zu den Wandungen (80) und (82) ist.

Selbstverständlich steht die Dichtung (66) mit ihrem äußeren Abschnitt (68) radial über dem Kolben (74) vor, um ein dichtendes Anliegen an der nicht dargestellten Zylinderinnenwandung der Spritze sicherzustellen.

Der innere Abschnitt (72) bewirkt ein Verstärken des membran- oder hautförmigen mittleren Abschnitts (70), ohne daß hierdurch Rückstellkräfte hervorgerufen werden könnten, die beim Injizieren bzw. Aspirieren hinderlich sind.

Dadurch, daß dein innere Abschnitt (72) mit seiner runden Außenfläche auf dem Boden (78) der Aufnahme (76) anliegt, erfolgt einerseits ein linienförmiges Abdichten gegenüber dem Boden (78) und andererseits eine rollende Bewegung beim Aspirieren bzw. Injizieren, durch die ein merklicher Reibungswiderstand nicht aufgebaut wird.

Gleiches gilt in bezug auf den äußeren Abschnitt (68), der gleichfalls in etwa linienförmig an der Zylinderinnenwandung anliegt und zum Abdichten eine rollende Bewegung entlang der Innenfläche ausübt, um dichtend an der Wandung (80) (Injektion) bzw. an der Wandung (82) (Aspiration) anzuliegen.

Zu den Abmessungen ist anzumerken, daß die radiale Ersteckung R₁ des äußeren-Abschnitts (68) in etwa gleich der radialen Erstreckung R₂ des mittleren Abschnitts(70) sein sollte. Dahingehend sollte die radiale Erstreckung R₃ des inneren Abschnitts (84) in etwa die Hälfte der des mittleren Abschnitts (70) bzw. des äußeren Abschnitts (68) sein. Der Radius R_{K} des Kolbens im Bodenbereich der Aufnahme (76) sollte dagegen in etwa gleich oder etwas größer als die Summe der radialen Erstreckungen des inneren Abschnitts (72) und des mittleren Abschnitts (70) sein.

## Patentansprüche

1. Sowohl zum Injizieren als auch zum Aspirieren geeignete Spritze mit in einem Zylinder (10, 36) verschiebbaren Kolben (74) sowie einer zwischen dem Kolben und Zylinderinnenwandung (26, 58) vorhandenen Dichtung (66), die zwischen zwei sich in Richtung der Zylinderinnenwandung (26, 58) erstreckenden Abschnitten (16, 20) des Kolbens oder einer umlaufenden Aufnahme (76) in dem Kolben angeordnet ist und in Längsrichtung des Kolbens betrachtet eine maximale Dicke aufweist, die kleiner oder gleich dem lichten Abstand (A) zwischen den Abschnitten des Kolbens bzw. den Begrenzungen (80, 82, 84, 86) der Aufnahme im Bereich dei Dichtung ist und einen maximalen Außendurchmesser aufweist, der zumindest gleich dein Innendurchmesser des Zylinders ist, wobei sich die Dichtung (66) aus einem mit den Abschnitten des Kolbens bzw. den Begrenzungen der Aufnahme und der Zylinderinnenwandung anliegenden äußeren, wulstartigen Abschnitt (46), einem den Kolben (74) dichtend umgebenden wulstartigen inneren Abschnitt (72) sowie einem membran- oder hautförmig ausgebildeten mittleren Abschnitt (70) über welchen der äußere und der innere Abschnitt ineinander übergehen und miteinander verbunden sind, zusammensetzt, wobei zwischen dem äußeren Abschnitt (68) der Dichtung und der Zylinderinnenwandung eine im wesentlichen linien förmige Abdichtung erfolgt,
**dadurch gekennzeichnet**,
daß die Dichtung (66) zwischen den Abschnitten des Kolbens (74) oder der in diesem vorhandenen Aufnahme (76) bei ruhender Spritze mit Spiel freibeweglich ist, daß der innere und der äußere Abschnitt (72, 68) der Dichtung (66) jeweils einen halbzylindrischen Abschnitt bilden und daß auch zwischen dem inneren Abschnitt (72) der Dichtung und dem Kolben (74) eine im wesentlichen linienförmige Abdichtung erfolgt.

2. Spritze nach Anspruch 1,
**dadurch gekennzeichnet**,
daß die Aufnahme (52, 76) für die Dichtung (44) eine umlaufende und im Schnitt V-förmige Vertiefung im Kolben (38, 74) ist.

3. Spritze nach Anspruch 1,
**dadurch gekennzeichnet**,
daß sich die Aufnahme (78) für die Dichtung (76) im Schnitt aus zwei symmetrisch zur Längsachse des Kolbens angeordnete rechteck- oder quadratförmige innere und trapezförmige äußere Abschnitte zusammensetzt.

4. Spritze nach zumindest einem der vorhergehenden Ansprüche,
**dadurch gekennzeichnet**,
daß der wulstartige äußere Abschnitt (46, 68) der Dichtung (44, 66) eine radiale Erstreckung (W, R₁) aufweist, die in etwa doppelt so groß wie die radiale Erstreckung (M, R₃) des inneren Abschnitts (48, 72) ist.

5. Spritze nach zumindest einem der vorhergehenden Ansprüche,
**dadurch gekennzeichnet**,
daß bei im Schnitt doppelhantelförmiger Dichtung (66) der zwischen den wulstartigen äußeren und inneren Abschnitten (68, 72) verlaufende haut- oder membranartige mittlere Abschnitt (70) eine radiale Erstreckung R₂ aufweist, die in etwa gleich der des äußeren Abschnitts (68) und in etwa doppelt so groß wie die des inneren Abschnitts (72) ist.

6. Spritze nach zumindest einem der vorhergehenden Ansprüche,
**dadurch gekennzeichnet**,
daß die Dichtung (24, 44) bei Nutzung der Spritze zum Injizieren dichtend zwischen der Zylinderinnenwandung (26, 58) und kolbenstirnseitig entferntliegendem Abschnitt (20) bzw. entferntliegender Begrenzung (62, 80, 86) und beim Aspirieren dichtend zwischen der Zylinderinnenwandung und kolbenstirnseitig naheliegendein Abschnitt (26) bzw. naheliegender Begrenzung (60, 82, 84) anliegt.

7. Spritze nach zumindest einem der vorhergehenden Ansprüche,
**dadurch gekennzeichnet**,
daß bei Verschieben des Kolbens (74) der äußere Abschnitt (68) und der innere Abschnitt (72) gegenüber der Zylinderinnenwandung bzw. dem Boden der Aufnahme (76) jeweils eine in etwa rollende Bewegung ausüben.

## Claims

1. A syringe adaptable to both injection and aspiration comprising a plunger (74) movable within a barrel (10, 36), and a gasket (66) provided between said barrel and the barrel inner wall (26, 58) disposed between two sections (16, 20) extending in the direction of said barrel inner wall (26, 58) of said plunger or an all-round receptacle (76) within the plunger and having a maximum thickness, seen in the longitudinal direction of said plunger, that is less than or equal to the inside distance (A) between said sections of said plunger or the limiting walls (80, 82, 84, 86) of said receptacle in the area of said gasket, respectively, and having a maximum outer diameter that is at least equal to the inner diameter of said barrel, said gasket (66) comprising an outer, bead-like section (46) adjacent said sections of said plunger or said limiting walls of said receptacle and said barrel inner wall, an inner, bead like section (72) surrounding said plunger (74) sealingly, as well as a membrane-like or skin-like central section (70), via which said outer and said inner section merge into one another and are connected to one another, wherein a substantially linear sealing effect is obtained between said outer section (68) of said gasket and said barrel inner wall,
**characterized in that**
said gasket (66) is freely movable with play between said sections of said plunger (74) or said receptacle (76) provided therein when the syringe is at rest, said inner and said outer section (72, 68) of said gasket (66) each form a semicylindrical section (70) and between the said section (72) of said gasket and said plunger (74) also a substantially linear sealing effect is obtained.

2. A syringe according to Claim 1,
**characterized in that**
said receptacle (52, 76) for said gasket (44) is an all-round depression of V-shaped section in said plunger (38, 74).

3. A syringe according to Claim 1,
**characterized in that**
said receptacle (78) for said gasket (76) comprises in section two rectangular or square inner sections and trapezoidal outer sections symmetrical in respect to the longitudinal axis of said plunger

4. A syringe according to at least one of the preceding claims,
**characterized in that**
said bead-like outer section (46, 68) of said gasket (44, 66) has a radial extent (W, R₁) that is approximately twice as large as the radial extent (M, R₃) of said inner section (48, 72).

5. A syringe according to at least one of the preceding claims,
**characterized in that**
when said gasket (66) is double-dumbbell-shaped in section said skin-like or membrane-like central section between said bead-like outer or inner sections (68, 72) has a radial extent R₂ that is approximately equal to that of said outer section (68) and approximately twice as great as that of said inner section (72).

6. A syringe according to at least one of the preceding claims,
**characterized in that**
said gasket (24, 44) is in sealing contact between said barrel inner wall (26, 58) and said section (20) distant from the end face of said plunger or the distant limiting wall (62, 80, 86) when said syringe is used for injection, and in sealing contact between said barrel inner wall and said section (26) proximal to the end face of said plunger or the proximal limiting wall (60, 82, 84) when said syringe is used for sucking.

7. A syringe according to at least one of the preceding claims,
**characterized in that**
when said plunger (84) is moved said outer section (68) and said inner section (72) exercise an approximately rolling movement in relation to the barrel inner wall and to the bottom of said receptacle, respectively.

## Revendications

1. Seringue utilisable aussi bien pour l'injection que pour l'aspiration avec un piston (74) déplaçable dans un cylindre (10, 36) ainsi qu'une garniture d'étanchéité (66) entre le piston et la paroi intérieure (26, 58) du cylindre, disposée entre deux secteurs (16, 20) du piston s'étendant en direction de la paroi intérieure (26, 58) du cylindre ou dans un logement circulaire (76) dans le piston et présentant, vu dans le sens longitudinal du piston, une épaisseur maximale inférieure ou égale à l'écartement (A) entre les secteurs du piston ou les limitations (80, 82, 84, 86) du logement dans la zone de la garniture d'étanchéité et présentant un diamètre extérieur maximal au moins égal au diamètre intérieur du cylindre, le joint d'étanchéité (66) étant composé d'un secteur (46) extérieur, en bourrelet, adjacent aux secteurs du piston ou aux limitations du logement et de la paroi intérieure du cylindre, d'un secteur (72) intérieur, en bourrelet, entourant de façon étanche le piston (74), ainsi qu'un secteur médian (70), en forme de membrane ou de pellicule, par-dessus duquel le secteur extérieur et le secteur intérieur se confondent et sont enliés entre eux, un étanchement essentiellement linéaire s'effectuant ainsi entre le secteur extérieur (68) de la garniture d'étanchéité et la paroi intérieure du cylindre,
**caractérisé en ce que**
la garniture d'étanchéité (66) est librement déplaçable entre les secteurs du piston (74) ou le logement (76) disponible, si la seringue est au repos et a du jeu, que le secteur intérieur et extérieur (72, 68) de la garniture d'étanchéité (66) forment respectivement un secteur demicylindrique et qu'un étanchement essentiellement linéaire s'effectue également entre le secteur intérieur (72) de la garniture et du piston (74).

2. Seringue selon la revendication 1,
**caractérisé en ce que**
le logement (52, 76) pour la garniture d'étanchéité (44) consiste en un évidement circulaire dans le piston (38, 74) et en coupe en forme de V.

3. Seringue selon la revendication 1,
**caractérisé en ce que**
le logement (78) pour la garniture d'étanchéité (76) se compose en coupe d'un secteur intérieur rectangulaire ou carré et d'un secteur extérieur trapézoïdal symétriques à l'axe longitudinal du piston.

4. Seringue selon au moins l'une des revendications précédentes,
**caractérisé en ce que**
le secteur extérieur en bourrelet (46, 68) de la garniture d'étanchéité (44, 66) présente une étendue radiale (W, R₁) d'environ deux fois la grandeur de l'étendue radiale (M, R₃) du secteur intérieur (48, 72).

5. Seringue selon au moins l'une des revendications précédentes,
**caractérisé en ce que**
sur la garniture d'étanchéité (66) en coupe en forme de double haltère le secteur médian (70) en forme de pellicule ou de membrane s'étendant entre les secteurs extérieurs en bourrelet et les secteurs intérieurs (68, 72) présente une étendue radiale R₂ environ égale à celle du secteur extérieur (68) et environ le double de celle du secteur intérieur (72).

6. Seringue selon au moins l'une des revendications précédentes
**caractérisé en ce que**
la garniture d'étanchéité (24, 44) est, en utilisant la seringue pour l'injection, en contact étanche entre la paroi intérieure (26, 58) du cylindre et le secteur (20) le plus éloigné du côté frontal du piston ou la limitation (62, 80, 86) la plus éloignée et pour l'aspiration en contact étanche entre la paroi intérieure du cylindre et le secteur (26) le plus proche du côté frontal du piston ou la limitation (60, 82, 84) la plus proche.

7. Seringue selon au moins l'une des revendications précédentes
**caractérisé en ce que**
pendant le déplacement du piston (74), le secteur extérieur (68) et le secteur intérieur (72) exercent un quasi mouvement de roulement par rapport à la paroi intérieure du cylindre ou le fond du logement (76).
